(19) Europäisches Patentamt
European Patent Office
Office européen des brevets

(11) Veröffentlichungsnummer: **0 081 090**
**B1**

(12) # EUROPÄISCHE PATENTSCHRIFT

(45) Veröffentlichungstag der Patentschrift: **05.08.87**

(51) Int. Cl.⁴: **C 07 D 309/32**, C 07 C 69/716

(21) Anmeldenummer: **82110275.3**

(22) Anmeldetag: **08.11.82**

(54) **Verfahren zur Herstellung von 3,4-Dihydro-alpha-pyronen.**

(30) Priorität: **05.12.81 DE 3148153**

(43) Veröffentlichungstag der Anmeldung: **15.06.83 Patentblatt 83/24**

(45) Bekanntmachung des Hinweises auf die Patenterteilung: **05.08.87 Patentblatt 87/32**

(84) Benannte Vertragsstaaten: **AT BE CH DE FR GB IT LI LU NL SE**

(56) Entgegenhaltungen:
**EP - A - 0 031 932**
**US - A - 4 235 780**

**Die Akte enthält technische Angaben, die nach dem Eingang der Anmeldung eingereicht wurden und die nicht in dieser Patentschrift enthalten sind.**

(73) Patentinhaber: **DYNAMIT NOBEL AKTIENGESELLSCHAFT, Postfach 1209, D-5210 Troisdorf, Bez. Köln (DE)**

(72) Erfinder: **Schmidt, Hans-Georg, Dr., Porzer Strasse 11, D-5216 Niederkassel-Ranzel (DE)**

**EP 0 081 090 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der vorliegenden Erfindung ist ein Verfahren 10 zur Herstellung von α-Pyronen der Formel

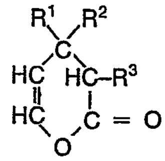

I,

in der $R^1$, $R^2$ und $R^3$ für gleiche oder verschiedene Alkylreste mit 1 bis 10 C-Atomen oder für Wasserstoff stehen.

Die Herstellung von α-Pyronen der oben genannten Formel wird z.B. in der DE-OS 29 52 068 beschrieben. Als Ausgangsprodukte werden dabei die in 3- und 4-Stellung entsprechend substituierten 5-Hydroximethyl-2(3H)-furanone eingesetzt, die einer thermischen Behandlung bei Temperaturen zwischen 200 und 500 °C unterworfen werden. Der Nachteil dieser Verfahrensweise besteht darin, dass diese Furanone in einem zusätzlichen Verfahrensschritt aus den entsprechenden 4,5-Epoxipentancarbonsäureestern gewonnen werden müssen, wodurch diese Arbeitsweise sehr aufwendig wird.

Es bestand deshalb die Aufgabe, ein Verfahren zur Herstellung von 3,4-Dihydro-α-pyronen aus 4,5-Epoxipentancarbonsäureestern zu finden, bei dem man in einer Stufe direkt das gewünschte Pyron erhält. In Erfüllung dieser Aufgabe wurde nun ein Verfahren zur Herstellung von 3,4-Dihydro-α-pyronen der oben genannten Formel gefunden, das dadurch gekennzeichnet ist, dass man eine Verbindung der allgemeinen Formel

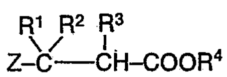

, II

in der $R^1$, $R^2$ und $R^3$ für Wasserstoff und/oder gleiche oder verschiedene Alkylreste mit 1 bis 10 C-Atomen und $R^4$ für Wasserstoff oder Alkylgruppe mit 1 bis 4 C-Atomen stehen und Z für die Gruppierung

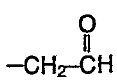 oder 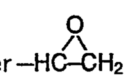

steht gegebenenfalls in Gegenwart eines Katalysators, auf Temperaturen zwischen 150 und 600 °C erhitzt.

Beim Einsatz von 4,5-Epoxipentancarbonsäurederivaten als Ausgangsprodukte, d.h. wenn in der oben genannten Formel Z für

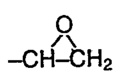

steht – entstehen bei dieser Arbeitsweise als Zwischenprodukte 4-Formylbuttersäurederivate, d.h. Verbindungen der oben genannten Formel II, bei denen Z für 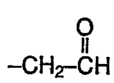 steht. Diese Verbindungen können gegebenenfalls isoliert werden und anschliessend zu solchen Insektiziden verarbeitet werden, die in der DE-OS 28 10 031 genannt sind. Zur Herstellung dieser Formylbuttersäurederivate war bisher nur die in der DE-OS 28 10 031 beschriebene Verfahrensweise bekannt, so dass in Erfüllung der oben genannten Aufgabe auch ein neues Verfahren zur Herstellung von 4-Formylbuttersäurederivaten gefunden wurde, das dadurch gekennzeichnet ist, dass man Epoxipentancarbonsäurederivate der Formel

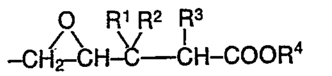

,

in der $R^1$, $R^2$, $R^3$ und $R^4$ die oben genannte Bedeutung haben, auf Temperaturen zwischen 200 und 600 °C, vorzugsweise zwischen 350 und 450 °C erhitzt.

Die Ausbeuten an diesem Zwischenprodukt hängen von der gewählten Temperatur und der Zeit des Erhitzens ab. Im allgemeinen erhält man gute Ausbeuten an Formylbuttersäurederivaten, wenn die Temperaturen im Bereich zwischen 350 und 450 °C liegen.

Erhitzt man die Verbindungen der allgemeinen Formel II in Gegenwart von silikatischen Materialien oder Aluminiumoxid, dann reagieren beide Verbindungsklassen der Formel II, also der Aldehyd und das Epoxid direkt zu Dihydropyronen der allgemeinen Formel I.

Als Substanzen, die die Thermolyse zur Bildung der 3,4-Dihydro-α-pyrone positiv beeinflussen, eignen sich oberflächenreiche, silikatische Materialien, wie z.B. wasserhaltige Alkali- oder Erdalkalialumosilikate. Von diesen eignen sich besonders gut die natürlichen und synthetischen Zeolithe und Montmorillonite. Letztere können auch z.B. durch Salzsäure nachbehandelt und auf Temperaturen zwischen 400 und 800 °C erhitzt sein, ehe sie erfindungsgemäss eingesetzt werden. Ein solcher nachbehandelter Montmorillonit besteht überwiegend (d.h. zu mehr als 90%) aus $SiO_2$ und enthält nur noch geringe Mengen an Aluminium, die im allgemeinen unter 1 Gew.-% liegen. Er enthält jedoc noch gebundenes Wasser, dessen Anteil zwischen 1 und 5 Gew.% schwanken kann. Seine Porosität liegt zwischen 55 und 65 Vol.-% (bestimmt durch Wasseraufnahme und Auftriebswägung gemäss DIN 51 056).

Auch getrocknete, poröse Kieselsäuregele oder Aluminiumoxid können als reaktionsbeschleunigende Substanzen eingesetzt werden.

Die erfindungsgemässe Thermolyse sowohl zur Herstellung der 3,4-Dihydro-α-pyrone als auch zur Herstellung der Formylbuttersäurederivate wird zweckmässigerweise so durchgeführt, dass man die umzusetzenden Verbindungen durch ein Rohr hindurch tropfen lässt, das auf die gewünschte Thermolysetemperatur erhitzt wird. Das Rohr ist mit temperaturstabilem inertem Material gefüllt, das die Reaktion nicht beeinflusst, wie z.B. Porzellan-Sättel oder Glaskugeln. Die Verweilzeit des Reaktionsgemischs in dem Rohr kann durch Füllhöhe, Form und Volumen dieser Körper weitgehend variiert werden.

Wenn erfindungsgemäss überwiegend die Herstellung von 3,4-Dihydro-α-pyronen gewünscht wird, können die temperaturstabilen, inerten Materialien teilweise oder vollständig durch Formkörper aus den oben genannten reaktionsbeschleunigenden Substanzen ersetzt werden. In diesem Fall wird die Umsetzung bevorzugt im Temperaturbereich zwischen 200 und 350 °C durchgeführt.

Im Rahmen der vorliegenden Erfindung ist es auch möglich, die Thermolyse der jeweiligen Ausgangsprodukte in Lösung durchzuführen, wobei als Lösungsmittel solche Verbindungen in Frage kommen, die bei den anzuwendenden Temperaturen stabil sind und mit keiner der Reaktionskomponenten reagieren. Beispiele für solche Lösungsmittel sind Benzol, Toluol und die Xylole. Diese Lösungsmittel können auch Siedepunkte besitzen, die unterhalb der gewählten Thermolysetemperatur liegen: Man tropft dann z.B. eine Lösung der Ausgangsprodukte in diesem Lösungsmittel durch ein senkrecht stehendes, oben beschriebenes Reaktionsrohr, wobei das Lösungsmittel verdampft und in einem Auffanggefäss gemeinsam mit den Reaktionsprodukten kondensiert wird.

Die Thermolyse lässt sich sowohl bei Atmosphärendruck als auch bei einem Unterdruck bis herab zu etwa $10^{-3}$ bar durchführen.

Die erfindungsgemäss herstellbaren 3,4-Dihydro-α-pyrone sind wertvolle Zwischenprodukte, die u.a. zur Herstellung von Insektiziden auf Basis von Cyclopropancarbonsäurederivaten Verwendung finden.

Beispiel 1

20 g 4,5-Epoxi-3,3-dimethylpentancarbonsäuremethylester wurden durch ein senkrecht angeordnetes Glasrohr (Länge 30 cm, Durchmesser 2,2 cm) innerhalb 160 min getropft. Das Glasrohr war mit Kugeln aus einem Montmorillonit gefüllt, der vorher während 8 Stunden mit 10%iger Salzsäure behandelt, anschliessend mit Wasser ausgewaschen und daraufhin für eine Stunde auf 500 °C erhitzt worden war. Die Innentemperatur des Rohres betrug 300 °C. Das Thermolysat wurde in einer gekühlten Vorlage aufgefangen und destilliert. Es wurden 15,3 g (97,5%) 3,4-Dihydro-4,4-dimethyl-α-pyron (Sdp.: 76–79 °C/16.$10^{-3}$ bar) erhalten.

Beispiel 2

37,6 g 4-Formyl-3,3-dimethylbuttersäuremethylester wurden durch ein senkrecht angeordnetes Glasrohr (Länge 30 cm, Durchmesser 2,2 cm), welches mit den in Beispiel 1 genannten Montmorillonitkugeln gefüllt war, innerhalb 240 min getropft. Die Innentemperatur des Rohres betrug 300 °C. Das Thermolysat wurde in einer gekühlten Vorlage aufgefangen und destilliert. Es wurden 28,8 g (97,6%) 3,4-Dihydro-4,4-dimethyl-α-pyron erhalten.

Beispiel 3

Analog zu Beispiel 2 werden 20 g 4-Formyl-3,3-dimethylbuttersäure zur Umsetzung gebracht. Es wurden 15 g (87,1%) 3,4-Dihydro-4,4-dimethyl-α-pyron erhalten.

Beispiel 4

70 g 4,5-Epoxi-3,3-dimethylpentancarbonsäuremethylester wurden durch ein senkrecht angeordnetes Quarzrohr (Länge 30 cm, Durchmesser 2,2 cm), welches mit Porzellan-Sätteln gefüllt war, innerhalb 560 min getropft. Die Innentemperatur des Quarzrohres betrug 400 °C. Das Thermolysat wurde in einer gekühlten Vorlage aufgefangen und bei einem Druck von 16 bar destilliert. Es wurden 57,7 g 4-Formyl-3,3-dimethylbuttersäuremethylester (Sdp. 84–88 °C/16.$10^{-3}$ bar) erhalten.

NMR-Spektrum (100 MHZ, $CCl_4$):$\delta$ = 1,13 (6H); 2,37 (2H); 2,47 (2H); 3,63 (3H); 9,78 (1H).

**Patentansprüche**

1. Verfahren zur Herstellung von 3,4-Dihydro-α-pyronen der Formel

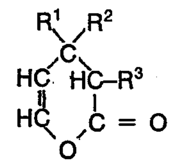

in der $R^1$, $R^2$ und $R^3$ für Wasserstoff und/oder Alkylgruppen mit 1 bis 10 C-Atomen stehen, dadurch gekennzeichnet, dass man eine Verbindung der Formel

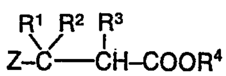

in der $R^1$, $R^2$ und $R^3$ die oben genannte Bedeutung haben, $R^4$ für Wasserstoff und/oder Alkylgruppen mit 1 bis 4 C-Atomen und Z für die Reste

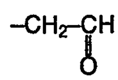 oder 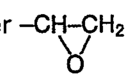

steht, gegebenenfalls in Gegenwart eines Katalysators, auf Temperaturen zwischen 150 und 600 °C erhitzt.

2. Verfahren gemäss Anspruch 1, dadurch gekennzeichnet, dass man die Ausgangsverbindung auf Temperaturen zwischen 250 und 350 °C erhitzt.

3. Verfahren gemäss Anspruch 1 oder 2, dadurch gekennzeichnet, dass das Erhitzen in Gegenwart von oberflächenreichen silikatischen Materialien oder Aluminiumoxid durchgeführt wird.

4. Verfahren gemäss einem der Ansprüche 1 bis 3, dadurch gekennzeichnet, dass man als oberflächenreiches silikatisches Material Zeolith oder Montmorillonit einsetzt.

5. Verfahren gemäss Anspruch 4, dadurch gekennzeichnet, dass man einen Montmorillonit einsetzt, der mit Salzsäure ausgewaschen und anschliessend einer thermischen Behandlung zwischen 400 und 800 °C unterworfen wurde.

6. Verfahren zur Herstellung der gemäss Anspruch 1 als Ausgangsprodukte einsetzbaren Formylbuttersäurederivate der Formel

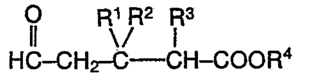 ,

in der $R^1$, $R^2$, $R^3$ und $R^4$ die oben genannte Bedeutung haben, dadurch gekennzeichnet, dass man ein Epoxid der Formel

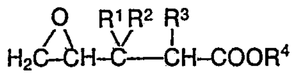 ,

in der $R^1$, $R^2$, $R^3$ und $R^4$ die oben genannte Bedeutung haben, auf Temperaturen zwischen 200 und 600 °C, vorzugsweise zwischen 350 und 450 °C erhitzt.

**Claims**

1. Process for the preparation of 3,4-dihydro-α-pyrones of the formula

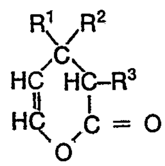

in which $R^1$, $R^2$ and $R^3$ stand for hydrogen and/or alkyl groups with 1 to 10 C-atoms, characterised in that a compound of the formula

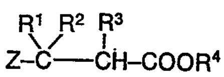

in which $R^1$, $R^2$ and $R^3$ have the above indicated meaning, $R^4$ stands for hydrogen and/or alkyl groups with 1 to 4 C-atoms and Z for the residues

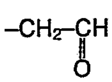 or 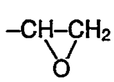

is heated to temperatures between 150 and 600 °C, optionally in the presence of a catalyst.

2. Process according to claim 1, characterised in that the starting compound is heated to temperatures between 250 and 350 °C.

3. Process according to claim 1 or 2, characterised in that the heating is carried out in the presence of silicate materials or aluminium oxide of large surface area.

4. Process according to one of claims 1 to 3, characterised in that zeolite or montmorillonite is employed as silicate material of large surface area.

5. Process according to claim 4, characterised in that a montmorillonite is employed which was subjected to washing out with hydrochloric acid and then to a thermal treatment at between 400 and 800 °C.

6. Process for the preparation of the formylbutyric acid derivatives of the formula

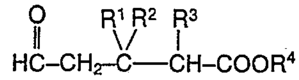 ,

in which $R^1$, $R^2$, $R^3$ and $R^4$ have the above-indicated meaning, which are employable as starting materials according to claim 1, characterised in that an epoxide of the formula

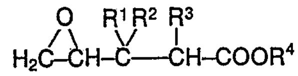 ,

in which $R^1$, $R^2$, $R^3$ and $R^4$ have the above-indicated meaning, is heated to temperatures between 200 and 600 °C, preferably between 350 and 450 °C.

**Revendications**

1. Procédé de préparation de 3,4-dihydro-alphapyrones de formule:

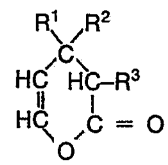

(dans laquelle $R^1$, $R^2$ et $R^3$ représentent chacun un atome d'hydrogène et/ou des groupes alkyles ayant 1 à 10 atomes de carbone), procédé caractérisé en ce qu'on chauffe à des températures comprises entre 150 et 600 °C, éventuellement en présence d'un catalyseur, un composé de formule:

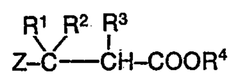

dans laquelle $R^1$, $R^2$ et $R^3$ ont le sens précité; $R^4$ représente un atome d'hydrogène et/ou des groupes alkyles ayant 1 à 4 atomes de carbone et Z représente les restes:

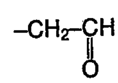 ou 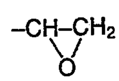

2. Procédé selon la revendication 1, caractérisé en ce qu'on chauffe jusqu'à des températures comprises entre 250 et 350 °C le composé de départ.

3. Procédé selon la revendication 1 ou 2, caractérisé en ce qu'on conduit le chauffage en présence de matières silicatées ayant une grande surface spécifique ou en présence d'oxyde d'aluminium.

4. Procédé selon l'une des revendications 1 à 3, caractérisé en ce qu'on utilise, comme matières silicatées ayant une grande surface spécifique, une zéolite ou de la montmorillonite.

5. Procédé selon la revendication 4, caractérisé en ce qu'on utilise une montmorillonite, qui a été extraite par lavage à l'acide chlorhydrique puis a été soumise à un traitement thermique effectué entre 400 et 800 °C.

6. Procédé pour préparer selon la revendication 1 des dérivés de l'acide formylbutyrique, utilisable à titre de produit de départ et répondant à la formule:

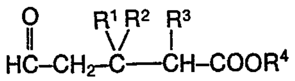

,

(dans laquelle $R^1$, $R^2$, $R^3$ et $R^4$ ont le sens précité), procédé caractérisé en ce qu'on chauffe jusqu'à des températures comprises entre 200 et 600 °C, avantageusement entre 350 et 450 °C, un époxyde de formule:

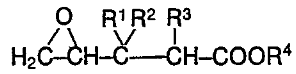

,

dans laquelle $R^1$, $R^2$, $R^3$ et $R^4$ ont le sens indiqué ci-dessus.